# EUROPÄISCHE PATENTANMELDUNG

(11) **EP 1 245 191 A2**
(43) Veröffentlichungstag der Anmeldung: **02.10.2002**
(21) Anmeldenummer: 02100304.1
(22) Anmeldetag: 27.03.2002
(51) Int. Cl.: A61B 8/08, A61B 8/12

(54) **Verfahren und bildgebendes Ultraschallsystem zur Bestimmung der Position eines Katheters**

(30) Priorität: 28.03.2001 DE 10115341
(71) Anmelder: Philips Corporate Intellectual Property GmbH, 52066 Aachen (DE); Koninklijke Philips Electronics N.V., 5621 BA Eindhoven (NL)
(72) Erfinder: Solf, Torsten, 52066 Aachen (DE); Eck, Kai, 52066 Aachen (DE)
(74) Vertreter: Volmer, Georg, Dipl.-Ing.

(57) **Zusammenfassung**

Die Erfindung betrifft ein bildgebendes Ultraschallsystem, das aus einem Ultraschallwandler (1) und einer Bildverarbeitungseinheit (2) zur Aufnahme eines dreidimensionalen Ultraschallbildes aus dem Körper eines Patienten (11) sowie einem Katheter (9) zur Durchführung einer therapeutischen oder diagnostischen Maßnahme im Körper des Patienten besteht. Der Katheter (9) trägt neben üblichen Instrumenten für sein vorgesehene Aufgabe an seiner Spitze drei beabstandete Ultraschallempfänger (10a - c), mit deren Hilfe das Eintreffen von Abtastsignalen des Ultraschallwandlers (1) detektiert werden kann. Aus der Laufzeit der Abtastsignale lässt sich dann der Abstand zwischen dem Ultraschallwandler (1) und den Empfängern (10a - c) berechnen. Die Empfänger (10a - c) können somit räumlich lokalisiert werden, was es insbesondere erlaubt, aus den dreidimensionalen Ultraschalldaten zB. diejenige Ebene auszuwählen, welche alle drei Empfänger (10a - c) des Katheters enthält. Die Spitze des Katheters kann somit automatisch verfolgt und jederzeit auf einem Monitor (4) dargestellt werden, ohne dass hierfür das manuelle Nachführen des Ultraschallwandlers (1) erforderlich wäre.

## Beschreibung

Die Erfindung betrifft ein Verfahren zur Bestimmung der Position eines Katheters in einem von einem Ultraschallwandler aufgenommenen Ultraschallbild Ferner betrifft sie ein bildgebendes Ultraschallsystem zur Darstellung eines Körperbereiches, mit welchem das genannte Verfahren durchgeführt werden kann.

Die Positionsbestimmung beziehungsweise Lokalisierung eines in das Gefäßsystem eines Patienten vorgeschobenen Katheters findet nach dem Stand der Technik vorzugsweise mit Hilfe einer Röntgen-Fluoroskopie (Röntgendurchleuchtung) statt. Dies gilt insbesondere für Untersuchungen und therapeutische Eingriffe im kardiovaskulären Bereich. Die Röntgen-Fluoroskopie ist zwar in ihrer Anwendung verhältnismäßig einfach, sie hat jedoch den Nachteil einer Strahlenbelastung für den Patienten und das medizinische Personal.

Zur Vermeidung einer derartigen Strahlenbelastung ist es bekannt, die Verfolgung und Führung eines Katheters über ein bildgebendes Ultraschallsystem vorzunehmen. Derartige Systeme haben den zusätzlichen Vorteil eines Weichteilkontrastes. Wünschenswert ist dabei insbesondere der Einsatz von 3D-Ultraschallscannern, welche mit flächig ("2D") angeordneten Ultraschallwandlereeinheiten in Echtzeit Bildinformationen aus einem dreidimensionalen Körpervolumen gewinnen. Die angemessene und gut verständliche Darstellung der dreidimensionalen Information stellt allerdings noch ein Problem dar. Wenn eine zweidimensionale Bildebene aus dem gescannten Volumen ausgewählt und dargestellt wird, enthält diese in der Regel nicht den Katheter, so dass dessen Führung über einen solchen Bildschnitt nicht möglich ist. Bewegungen des Körpers durch zum Beispiel Atmung oder Herzschlag tragen zu einer weiteren Verkomplizierung bei. Der verwendete Ultraschallgeber muss daher in der Regel ständig manuell nachgeführt und ausgerichtet werden, um die interessierende Position des Katheters zu verfolgen.

Aus der US 4 706 681 ist ein Verfahren zur Bestimmung der Position der Spitze einer im Herzen angeordneten Stimulationselektrode bekannt, bei welchem in die genannte Spitze ein Ultraschallempfänger integriert ist. Dieser erzeugt ein elektrisches Signal, wenn er von dem fächerförmigen zweidimensionalen Abtaststrahl eines 1D-Ultraschallwandlers getroffen wird. Das Signal wird über den Elektrodendraht nach außen geleitet, wo es von einer Auswerteeinheit erkannt werden kann. Das Auftreten dieses Signals liefert somit einen Hinweis darauf, dass sich die Spitze der Elektrode gerade im Strahlungsbereich des Ultraschallwandlers befindet. Auf diese Weise ist es möglich, auf dem Ultraschallbild die Spitze der Elektrode von einem sonstigen Schnitt durch den Elektrodendraht zu unterscheiden. Eine über die Bildinformation des Ultraschallwandlers hinausgehende Positionsbestimmung der Elektrode ist jedoch nicht möglich.

Aus der WO 96/25881 ist ein Verfahren zur Verfolgung der Position eines Instrumentes im Körper eines Patienten bekannt, bei welchem das Operationsgebiet des Instrumentes mit einem bildgebenden Ultraschallsystem überwacht und dargestellt wird, wobei gleichzeitig über ein magnetisches Positionierungssystem die Lage des Instrumentes bestimmt wird. Bei einem starren Instrument kann so die Position des außerhalb seines Körpers gelegenen Endes festgestellt und daraus auf die Position der im Körper befindlichen Spitze geschlossen werden. In einem bildverarbeitenden System werden dann die Informationen des Positionierungssystems und des Ultraschallsystems in der Darstellung des Operationsgebietes kombiniert.

Weiterhin sind aus der US 5 954 649 und der WO 00/07501 Verfahren bekannt, bei denen ein oder mehrere Referenzkatheter mit Ultraschallsendern in den Körper eingeführt und in der Nähe des Operationsbereiches eines zu beobachtenden Katheters mit mindestens einem Ultraschallempfänger positioniert werden. Mit dieser Anordnung kann durch Laufzeitmessungen von Ultraschallsignalen die Position des Katheters relativ zu den Referenzkathetern bestimmt werden. Nachteilig ist jedoch der Aufwand für den zusätzlichen Einsatz der Referenzkatheter sowie die Tatsache, dass die Position nur relativ zu den Referenzkathetern bestimmt wird, deren Lokalisation wiederum nicht exakt bekannt ist.

Vor diesem Hintergrund war es Aufgabe der vorliegenden Erfindung, ein Verfahren und ein System zur Darstellung eines Katheters im Körper eines Patienten bereitzustellen, die für den Patienten schonend sind und gleichzeitig mehr Informationen für den Anwender bereitstellen.

Diese Aufgabe wird durch ein Verfahren mit den Merkmalen des Anspruchs 1 und ein bildgebendes System mit den Merkmalen des Anspruchs 8 gelöst. Vorteilhafte Ausgestaltungen sind in den Unteransprüchen enthalten.

Das Verfahren dient der Bestimmung der Position eines Katheters in einem von einem Ultraschallwandler aufgenommenen Ultraschallbild, wobei der Ultraschallwandler in bekannter Weise mindestens ein Sendeelement zum Aussenden von Ultraschall-Abtastsignalen und mindestens ein Empfangselement zum Empfangen von aus dem Körper zurückkehrenden Echos des Abtastsignals aufweist. Sende- und Empfangselement(e) können gegebenenfalls auch dasselbe Element sein. Weiterhin besitzt bei dem Verfahren der Katheter mindestens einen Ultraschallempfänger, der den Empfang von Ultraschall-Abtastsignalen, die vom Ultraschallwandler gesendet wurden, detektiert. Die Laufzeit der Ultraschall-Abtastsignale vom Sendeelement des Ultraschallwandlers zum Empfänger am Katheter wird dann gemessen, und aus dieser so bestimmten Laufzeit wird der Abstand zwischen dem Ultraschallwandler bzw. dem Sendeelement und dem Empfänger am Katheter ermittelt.

Das geschilderte Verfahren hat den Vorteil, dass es eine genauere Positionsbestimmung des Katheters relativ zum meistens außerhalb des Körpers befindlichen Ultraschallwandlers erlaubt, da es den jeweiligen Abstand zwischen diesen beiden Elementen bestimmt. Die so erhaltene Information kann dann verwendet werden, um in einem Ultraschallbild den interessierenden Katheter genauer zu lokalisieren.

Vorzugsweise ist der Ultraschallwandler dabei so ausgestaltet, dass er ein dreidimensionales Ultraschallbild aufnimmt. Ein solcher Wandler hat eine Mehrzahl von Sendeeinheiten, so dass der Abstand des Ultraschallempfängers bzw. der Ultraschallempfänger am Katheter zu jeder der Sendeeinheiten bestimmt werden kann. Auf diese Weise ist es möglich, die genaue räumliche Position jedes Ultraschallempfängers am Katheter zu bestimmen.

Bei der Verwendung eines Ultraschallwandlers, der ein dreidimensionales Bild aufnimmt, erlaubt das vorgeschlagene Verfahren aufgrund der damit ermittelbaren Position des Ultraschallempfängers am Katheter, dass in Abhängigkeit von der Position dieses Ultraschallempfängers eine Ebene aus dem dreidimensionalen Ultraschallbild ausgewählt und dargestellt wird. Insbesondere kann es sich dabei um eine Ebene handeln, welche den Ultraschallempfänger und daher auch den Katheter enthält. Es ist somit nicht mehr erforderlich, von Hand den Ultraschallwandler beziehungsweise die aus dem dreidimensionalen Ultraschallbild dargestellte Ebene dem Katheter nachzuführen, sondern diese Ebene kann mit Hilfe der Position des Ultraschallempfängers am Katheter automatisch gefunden werden.

Vorzugsweise enthält der Katheter mindestens drei Ultraschallempfänger, so dass sich die räumliche Position von drei verschiedenen Punkten des Katheters ermitteln lässt. Diese drei Punkte definieren dann eine Ebene, durch die der Katheter verläuft. Wenn diese Ebene aus einem dreidimensionalen Ultraschallbild zur Darstellung ausgewählt wird, so ist sichergestellt, dass sie den Katheter über einen längeren Verlaufsabschnitt enthält. Für den Ausnahmefall, dass alle Ultraschallempfänger am Katheter in einer Linie ausgerichtet sind und sie daher keine eindeutige Ebene definieren, kann willkürlich eine derjenigen Ebenen zur Darstellung ausgewählt werden, welche alle drei Ultraschallempfänger enthalten.

Bei der Darstellung des aufgenommenen Ultraschallbildes, die zum Beispiel auf einem Monitor erfolgen kann, wird vorzugsweise die aus den Laufzeitmessungen bekannte Position des Empfängers oder der Empfänger am Katheter hervorgehoben angezeigt. Dies liefert dem Anwender eine Information darüber, wo die Empfänger, deren Position exakt bekannt ist, liegen.

Gemäß einer anderen Weiterbildung des Verfahrens wird mit Hilfe der ermittelten Position der Ultraschallempfänger des Katheters ein darzustellender Unterbereich des gesamten Ultraschall-Abtastvolumens bestimmt. Die nachfolgende Ultraschalldarstellung kann sich dann auf diesen Unterbereich konzentrieren. Die Ermittlung der Position des Katheters erlaubt es somit, aus dem gesamten gescannten Körpervolumen eine "Region of Interest" (ROI) festzulegen. Die Beschränkung der Bildakquisition auf diese ROI ermöglicht dann eine erhebliche Einsparung von Zeit, die für höhere Bildakquisitionsraten, für Rauschreduktion, für Mulitfoci etc. verwendet werden kann.

Mit Hilfe des Katheters, dessen Position über die an ihm angeordneten Ultraschallempfänger bestimmt wird, werden vorzugsweise noch weitere Messdaten aufgenommen, die dann der ermittelten Position des Ultraschallempfängers zugeordnet werden können. Insbesondere kann es sich bei diesen Messdaten um elektrophysiologische Daten handeln, also zum Beispiel Reizleitungspotentiale am Herzen.

Die Erfindung betrifft ferner ein bildgebendes Ultraschallsystem zur Darstellung eines Körperbereiches, enthaltend die folgenden Elemente:
- einen Ultraschallwandler mit mindestens einer Sendeeinheit zum Senden von Ultraschall-Abtastsignalen und mit mindestens einer Empfangseinheit zum Empfangen von Echos der Abtastsignale;
- eine Bildverarbeitungseinheit, welche mit dem Ultraschallwandler gekoppelt und dazu eingerichtet ist, aus den vom Ultraschallwandler bereitgestellten Messsignalen eine Darstellung des Körperbereiches zu berechnen;
- mindestens einen Katheter mit mindestens einem Ultraschallempfänger, welcher mit der Bildverarbeitungseinheit gekoppelt ist.

Die Bildverarbeitungseinheit ist dabei dazu eingerichtet, aus der gemessenen Laufzeit der Abtastsignale von der Sendeeinheit des Ultraschallwandlers zu dem Empfänger oder zu den Empfängern des Katheters den Abstand zwischen der Sendeeinheit und dem/den Empfänger(n) zu bestimmen. Mit einem derartigen bildgebenden System ist es somit möglich, die Position des Katheters genauer festzulegen.

Das bildgebende System ist insbesondere so eingerichtet beziehungsweise so weitergebildet, dass mit ihm ein Verfahren der oben erläuterten Art durchgeführt werden kann.

Vorzugsweise enthält der Ultraschallwandler eine Mehrzahl von Ultraschall-Sendeeinheiten und Ultraschall-Emfangseinheiten, und er ist zusammen mit der Bildverarbeitungseinheit dazu eingerichtet, ein dreidimensionales Ultraschallbild aufzunehmen. Bei einem solchen Ultraschallwandler mit mehreren Sendeeinheiten kann der Abstand jeder Sendeeinheit zu jedem Empfänger am Katheter ermittelt werden. Die genaue Lage der Katheterempfänger im gescannten 3D-Volumen läßt sich aus dem bekannten Pulsgenerierungstiming des bildgebenden Systems und den Laufzeitmesungen der Katheterempfänger bestimmen.

Weiterhin weist der Katheter vorzugsweise mindestens drei Ultraschallempfänger auf, so dass die Position von drei verschiedenen Punkten des Katheters ermittelt werden kann. Diese drei Punkte können dann verwandet werden, um eine aus einem dreidimensionalen Ultraschallbild darzustellende Ebene festzulegen, wobei diese Ebene zumindest einen Abschnitt des Katheters enthält.

Weiterhin ist es bevorzugt, wenn der Katheter weitere medizinische Instrumente wie insbesondere Elektroden zur Aufnahme elektrophysiologischer Daten aufweist. Das heißt, dass es sich bei dem Katheter um ein bekanntes therapeutisches oder diagnostisches Instrument handeln kann, welches durch die zusätzliche Ausrüstung mit den Ultraschallempfängern für den Einsatz in einem Verfahren unter Ultraschallbeobachtung besonders geeignet ist.

Im Folgenden wird die Erfindung mit Hilfe der Figuren beispielhaft erläutert. Es zeigt:
- Fig. 1: schematisch die Komponenten eines bildgebenden Systems zur Bestimmung der Position eines Katheters;
- Fig. 2: die Spitze des in dem System nach Figur 1 verwendeten Katheters.

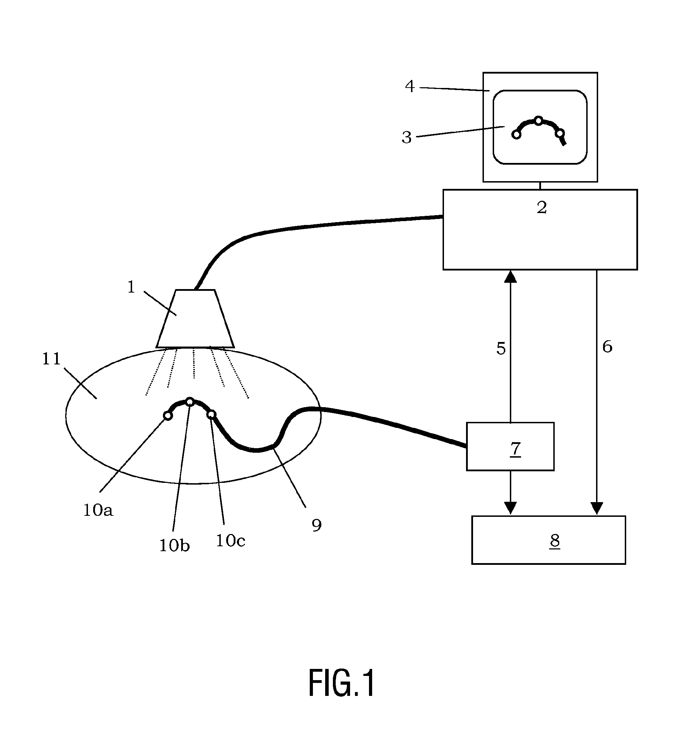

Das in Figur 1 dargestellte bildgebende System besteht im Wesentlichen aus einem 2D-Ultraschallwandler 1, der mit einer 3D-Bildverarbeitungseinheit 2 verbunden ist, sowie einem (Einmal-)Katheter 9, welcher an seiner Spitze drei Ultraschallempfänger 10a, 10b und 10c aufweist, die über eine Schnittstelle 7 ebenfalls mit der Bildverarbeitungseinheit 2 verbunden sind.

Der Ultraschallwandler 1 und die Bildverarbeitungseinheit 2 dienen in bekannter Weise dazu, aus dem im Einstrahlbereich der zweidimensional verteilten Sendeeinheiten des Ultraschallwandlers 1 befindlichen Körpervolumen eines Patienten 11 ein dreidimensionales Echobild zu gewinnen. Aus den so erhaltenen volumetrischen Ultraschalldaten kann dann auf einem Monitor 4, der mit der Bildverarbeitungseinheit 2 verbunden ist, eine ausgewählte Ebene 3 dargestellt werden. Ferner ist auch die Darstellung mehrerer Ebenen gleichzeitig oder - z.B. mit Hilfe einer 3D-Brille - einer dreidimensionalen Abbildung möglich.

Bei dem Katheter 9 kann es sich grundsätzlich um jede Art von Katheter handeln, welcher üblicherweise zur Behandlung oder Diagnose eingesetzt wird. Insbesondere kann der Katheter 9 für die Durchführung einer PTCA (Percutaneous Transluminal Coronary Angioplasty), einer Perfusion, eines elektrophysiologischen Mapping, einer Ablation, einer Blutdruckmessung oder dergleichen bestimmt sein. Weiterhin kann ein Herzwandkontakt mit dem erfindungsgemäßen System gut verfolgt werden.

Zur Beobachtung und Führung des Katheters 9 ist es wünschenswert, dass seine Spitze möglichst permanent auf dem Monitor 4 dargestellt wird. Während dies bei herkömmlichen Systemen nur durch die ständige manuelle Nachführung der Darstellungsebene erreicht werden kann, kann diese Ebene bei dem erfindungsgemäßen System automatisch erkannt und zur Darstellung auf dem Bildschirm 4 ausgewählt werden.

Um dies zu ermöglichen, sind an der Spitze des Katheters 9 beabstandet voneinander drei Ultraschallempfänger 10a, 10b und 10c angeordnet. Von diesen Empfängern können die vom Ultraschallsystem erzeugten Ultraschallimpulse detektiert werden. Die gemessenen Signale werden dann über die Schnittstelle 7 und eine Leitung 5 der Bildverarbeitungseinheit 2 mitgeteilt, welche aus den gemessenen Laufzeiten der empfangenen Impulse bei bekannter Pulsfolge und Geometrie des Ultraschallwandlers 1 in Echtzeit die Position jedes der drei Empfänger 10a, 10b, 10c berechnet. Die drei Ultraschallempfänger und damit die Spitze des Katheters 9 können somit relativ zum außerhalb des Körpers befindlichen Ultraschallwandler lokalisiert werden. Die Bildverarbeitungseinheit 2 kann dann die bekannten Positionen der Ultraschallempfänger 10a, 10b und 10c dazu verwenden, eine geeignete Darstellungsebene 3 aus den volumetrischen Ultraschalldaten auszuwählen und auf dem Monitor 4 darzustellen. Vorzugsweise wird es sich dabei um diejenige Ebene handeln, welche alle drei Ultraschallempfänger 10a, 10b und 10c enthält. In dem so erzeugten Bild können zusätzlich die Positionen der Empfänger hervorgehoben dargestellt werden.

Die automatische Auswahl der "richtigen", das heißt den Katheter 9 enthaltenden Ebene aus den volumetrischen Ultraschalldaten ermöglicht es, dass der Ultraschallwandler 1 während der gesamten Untersuchung ortsfest und gleich ausgerichtet bleiben kann, so dass keine Nachführung von Hand erforderlich ist.

Sollten sich die drei Ultraschallempfänger 10a,10b und 10c ausnahmsweise einmal in gerader Linie befinden, so definieren sie keine eindeutige Ebene. In diesem Falle wird z.B. diejenige der die drei Empfänger enthaltenden Ebenen ausgewählt, welche in eine vorgegebene Richtung weist.

Durch die mit dem vorgeschlagenen System mögliche automatische Positionsbestimmung des Katheters 9 ist es ferner möglich, aus dem gescannten Volumen einen Unterbereich zu isolieren und nicht interessierende Randbereiche in nachfolgenden Ultraschallaufnahmen nicht mehr zu berücksichtigen. Das Ultraschallverfahren kann sich somit automatisch auf eine ROI ("Region of Interest") konzentrieren, wodurch sich die erforderliche Aufnahmezeit verringern lässt und wodurch eine höhere Bildrate des Ultraschallsystems erzielt werden kann.

Über eine Ausgabeleitung 6 können die dreidimensionalen Positionsinformationen von der Bildverarbeitungseinheit 2 auch an ein anderes, insbesondere anwendungsspezifisches System 8 weitergeleitet werden. So kann etwa ein an dem Katheter 9 zusätzlich befindliches Instrument betätigt werden, wenn die Ultraschallempfänger 10a, 10b und 10c eine bestimmte Position erreicht haben.

Auch die Schnittstelle 7 ist zum Informationsaustausch über eine Leitung 7 mit dem anwendungsspezifischen System 8 verbunden. Über diese Leitung 7 können z.B. vom Katheter gemessene elektrophysiologische Signale weitergeleitet werden.

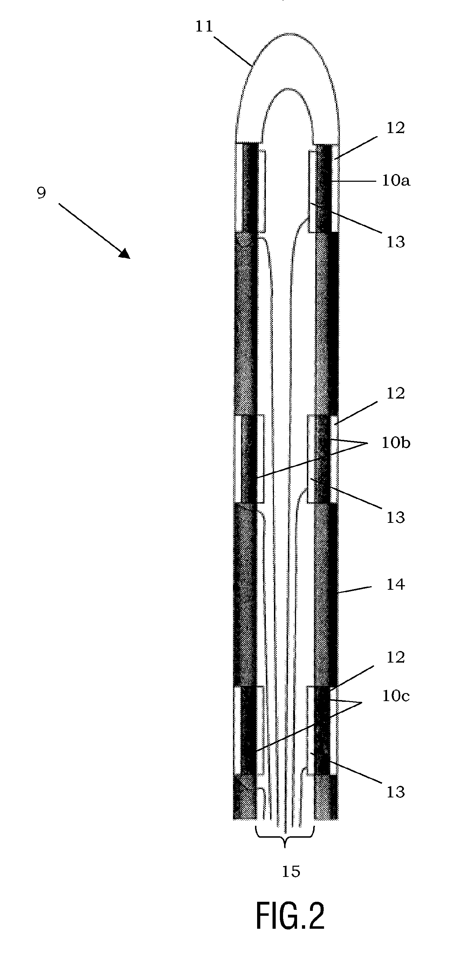

In Figur 2 ist eine mögliche Ausgestaltung des distalen Endes des Katheters 9 detaillierter dargestellt. Der Katheter 9 weist dabei eine abgerundete Spitze 11 auf, welche eine flexible Katheterröhre 15 abschließt. Entlang der Röhre 15 sind beabstandet voneinander drei Ringelektroden 12 vorgesehen, über welche elektrophysiologische Daten aufgezeichnet werden können. Unter den Ringelektroden 12 liegt jeweils ein ringförmiges piezoelektrisches Empfängerelement 10a, 10b und 10c. Dieses Piezoelement erzeugt ein elektrisches Signal, wenn es von einem Ultraschall-Abtastimpuls des Ultraschallwandlers 1 (Figur 1) getroffen wird.

Zum inneren Hohlraum des Katheters 9 hin wird das Ultraschallelement jeweils von einer inneren Elektrode 13 abgedeckt. Alle Elektroden 12, 13 sind an durch den Hohlraum des Katheters 9 führende Leitungen angeschlossen, welche in einem Leitungsbündel 15 zur Schnittstelle 7 (Figur 1) führen.

Der dargestellte Katheter eignet sich insbesondere für die Analyse von Herzarrhythmien. Dabei kann die dreidimensionale Positionsinformation für ein elektrophysiologisches Mapping hilfreich sein, da geometrische Informationen direkt mit den elektrischen Aktivitäten des Herzens überlagert werden können. Auf diese Weise kann eine anatomische Karte erzeugt werden, welche die Weiterleitung der elektrischen Erregung darstellt und die Identifikation der Ursache der Arrhythmie erlaubt.

## Patentansprüche

1. Verfahren zur Bestimmung der Position eines Katheters in einem von einem Ultraschallwandler aufgenommenen Ultraschallbild, wobei der Katheter mindestens einen Ultraschallempfänger aufweist, der den Empfang von Ultraschall-Abtastsignalen detektiert, und wobei aus der gemessenen Laufzeit der Abtastsignale der Abstand zwischen dem Ultraschallwandler und dem Ultraschallempfänger am Katheter ermittelt wird.

2. Verfahren nach Anspruch 1,
**dadurch gekennzeichnet,**
**dass** der Ultraschallwandler ein dreidimensionales Ultraschallbild aufnimmt, und dass aus der gemessenen Laufzeit der Abtastsignale die räumliche Position des Ultraschallempfängers am Katheter bestimmt wird.

3. Verfahren nach Anspruch 2,
**dadurch gekennzeichnet,**
**dass** in Abhängigkeit von der ermittelten Position des Ultraschallempfängers mindestens eine Ebene aus dem dreidimensionalen Ultraschallbild ausgewählt und dargestellt wird.

4. Verfahren nach Anspruch 3,
**dadurch gekennzeichnet,**
**dass** diejenige Ebene aus dem dreidimensionalen Ultraschallbild dargestellt wird, welche mindestens einen, vorzugsweise alle Ultraschallempfänger enthält, so dass die Position des Katheters fortlaufend verfolgt werden kann.

5. Verfahren nach Anspruch 1,
**dadurch gekennzeichnet,**
**dass** die Position des Ultraschallempfängers in einer Darstellung des aufgenommenen Ultraschallbildes hervorgehoben wird.

6. Verfahren nach Anspruch 1,
**dadurch gekennzeichnet,**
**dass** anhand der ermittelten Position des Ultraschallempfängers ein Unterbereich des Abtastvolumens bestimmt wird, auf welchen sich die nachfolgende Ultraschalldarstellung konzentriert.

7. Verfahren nach Anspruch 1,
**dadurch gekennzeichnet,**
**dass** mit Hilfe des Katheters weitere Messdaten, insbesondere elektrophysiologische Daten, aufgenommen und der ermittelten Position des Ultraschallempfängers zugeordnet werden.

8. Bildgebendes Ultraschallsystem zur Darstellung eines Körperbereiches, enthaltend
- einen Ultraschallwandler mit mindestens einer Sendeeinheit zum Senden von Ultraschall-Abtastsignalen und mit mindestens einer Empfangseinheit zum Empfangen von Echos der Abtastsignale;
- eine Bildverarbeitungseinheit, welche mit dem Ultraschallwandler gekoppelt und dazu eingerichtet ist, aus den Messsignalen des Ultraschallwandlers eine Darstellung des Körperbereiches zu berechnen;
- mindestens einen Katheter mit mindestens einem Ultraschallempfänger, welcher mit der Bildverarbeitungseinheit gekoppelt ist,
wobei die Bildverarbeitungseinheit dazu eingerichtet ist, aus der Laufzeit der Abtastsignale den Abstand zwischen der Sendeeinheit des Ultraschallwandlers und dem Ultraschallempfänger am Katheter zu bestimmen.

9. Bildgebendes Ultraschallsystem nach Anspruch 8,
**dadurch gekennzeichnet,**
**dass** der Ultraschallwandler eine Mehrzahl von Sendeeinheiten und Empfangseinheiten aufweist und zusammen mit der Bildverarbeitungseinheit dazu eingerichtet ist, ein dreidimensionales Ultraschallbild aufzunehmen.

10. Bildgebendes Ultraschallsystem nach Anspruch 8,
**dadurch gekennzeichnet,**
**dass** der Katheter mindestens drei Ultraschallempfänger aufweist.

11. Bildgebendes System nach Anspruch 8,
**dadurch gekennzeichnet,**
**dass** der Katheter weitere medizinische Instrumente, insbesondere Elektroden zur Aufnahme elektrophysiologischer Daten, aufweist.

12. Bildgebendes System nach Anspruch 8,
**dadurch gekennzeichnet,**
**dass** es dazu eingerichtet ist, ein Verfahren nach einem der Ansprüche 1 bis 7 auszuführen.
